# EUROPEAN PATENT SPECIFICATION

(11) **EP 3 804 613 B1**
(45) Date of publication and mention of the grant of the patent: **05.08.2026**
(21) Application number: 18921425.7
(22) Date of filing: 05.06.2018
(51) Int. Cl.: A61B 5/024, A61B 5/11, A61B 5/00, A61B 5/349

(54) **MOTION SENSOR-BASED PHYSIOLOGICAL PARAMETER OPTIMIZATION METHOD AND MONITORING DEVICE**
VERFAHREN ZUR OPTIMIERUNG PHYSIOLOGISCHER PARAMETER AUF BASIS VON BEWEGUNGSSENSOREN UND ÜBERWACHUNGSVORRICHTUNG
PROCÉDÉ D'OPTIMISATION DE PARAMÈTRE PHYSIOLOGIQUE FONDÉ SUR UN CAPTEUR DE MOUVEMENT ET DISPOSITIF DE SURVEILLANCE

(43) Date of publication of application: 14.04.2021
(73) Proprietor: Shenzhen Mindray Bio-Medical Electronics Co., Ltd., Shenzhen, Guangdong 518057 (CN); Shenzhen Mindray Scientific Co., Ltd., Shenzhen, Guangdong 518000 (CN)
(72) Inventor: LIU, Sanchao, Shenzhen, Guangdong 518057 (CN); MA, Qiang, Shenzhen, Guangdong 518057 (CN); SUN, Zehui, Shenzhen, Guangdong 518057 (CN); REN, Jian, Shenzhen, Guangdong 518057 (CN); HE, Xianliang, Shenzhen, Guangdong 518057 (CN); LIU, Qiling, Shenzhen, Guangdong 518057 (CN); JIN, Xingliang, Shenzhen, Guangdong 518057 (CN); YE, Zhigang, Shenzhen, Guangdong 518057 (CN); LUO, Hanyuan, Shenzhen, Guangdong 518057 (CN); ZHANG, Ningling, Shenzhen, Guangdong 518057 (CN)
(74) Representative: KIPA AB
(86) International application number: PCT/CN2018/089960
(87) International publication number: WO 2019/232695

(56) References cited:
- EP-A1- 1 724 684
- WO-A1-2017/140663
- CN-A- 105 380 630
- CN-A- 105 530 858
- CN-A- 105 726 016
- CN-A- 105 816 163
- CN-A- 107 466 222
- CN-A- 107 714 024
- US-A1- 2016 089 086
- US-A1- 2016 228 069

## Description

### Technical Field

The disclosure relates to the field of medical apparatuses, and in particular to a method for optimizing physiological parameter using a motion sensor and a monitoring device.

### Background

With the development of medical technologies and improvement of people's cognition of medicine, the importance and attention of rapid recovery after operation have been sharply enhanced and promoted. During the postoperative recovery period, medical personnel hope that patients can get out of bed and do more activities, to promote rapid physical recovery. A conventional bedside monitoring device limits an activity space of the patient, and the patient cannot do activities comfortably due to lengthy and complicated cables. When the patients do activities, a physiological signal of the patient may also be monitored, but the patient's activities may interfere with the monitored physiological signal, so that the physiological signal of the patient doing activities cannot be accurately monitored.

EP1724684A1 concerns a real-time operating system and a method of analysis of an ECG signal. The method comprises acquiring one or more ECG signals over a period of time; filtering the one or more ECG signals with at least one filter selected from the group consisting of a low pass filter, a high pass filter, and an adaptive filter, and extracting one or more features from the filtered signal.

US20160228069 discloses a processing apparatus for processing a physiological signal. The processing apparatus is configured to obtain physiological signal containing at least two wave cycles, obtaining a feature signal descriptive of occurrences of a feature in the physiological signa, determining an average waveform of the physiological signal, and determining a mode signal comprising amplitude-scaled instances of the average waveform of the physiological signal placed at the occurrences of the feature in the physiological signal.

CN105816163B provides a method for detecting a heart rate. The method is applied to a wearable device, and includes: calculating a time domain characteristic value and a frequency domain characteristic value of the first PPG data acquired in a preset time period; determining a heart rate signal quality in the preset time period according to the time domain feature value of the first PPG data, the frequency domain feature value, and the activity amount of the user in the preset time period; determining, according to the time domain feature value of the first PPG data, the frequency domain feature value, the heart rate signal quality in the preset time period, and heart rate related data of a previous preset time period, determining the user in the location The heart rate value in the preset time period, wherein the heart rate related data includes a heart rate value and a heart rate signal quality.

CN105726016B provides a method for outputting an electrocardiogram, including the following steps: collecting ECG data; analyzing and comparing the ECG data, and if the ECG data exceeds a preset range, performing corresponding over-labeling and output; extracting the ECG data marked beyond the standard and sending it to the hospital information system.

Therefore, the current monitoring device still has many limitations.

### Summary

In view of the above problems, the disclosure mainly provides a method for optimizing physiological parameter using a motion sensor and a monitoring device.

According to a first aspect, an embodiment provides a method for optimizing physiological parameter using a motion sensor as defined in claim 1, the method comprising:
acquiring a physiological signal of a monitored object by a physiological sensor attached to the monitored object, and synchronously acquiring a motion signal of the monitored object by a motion sensor attached to the monitored object; the physiological signal is an ECG signal acquired by an electrocardiogram sensor;
determining a signal characteristic of the physiological signal from the physiological signal, and determining whether the physiological signal is reliable according to the signal characteristic of the physiological signal; wherein the signal characteristic of the physiological signal comprises signal quality, matching of QRS complexes and an effectiveness of QRS complexes, and said determining whether the physiological signal is reliable according to the signal characteristic of the physiological signal comprises: assigning a weight to each signal characteristic of the physiological signal, acquiring a reliability score of the physiological signal through voting and scoring, and determining whether the physiological signal is reliable based on the reliability score;
when determining the physiological signal is reliable, directly calculating the physiological parameter of the monitored object based on the signal characteristic of the physiological signal;
when determining the physiological signal is unreliable, determining a signal characteristic of the motion signal from the motion signal, and determining a motion state by grading the motion comprising at least a low motion grade and a high motion grade, during each heartbeat period of the monitored object during a time period in which the motion signal is acquired according to the signal characteristic of the motion signal, and wherein the heartbeat period is a QRS complex interval; and
according to the motion state during the heartbeat period, determining an effectiveness of the physiological signal during the heartbeat period, wherein the signal is determined as effective based on the low motion grade and ineffective and/or invalid at the high motion grade, or correcting the signal characteristic of the physiological signal based on how the motion has been graded during the heartbeat period when calculating a physiological parameter; and
wherein the signal characteristic of the physiological signal and the motion signal is a time domain characteristic and/or a frequency domain characteristic of a signal; the time domain characteristic of the signal comprises: at least one of a peak position, a peak amplitude, a peak slope, a peak width, peak effectiveness, a peak type, a peak-to-peak interval value, peak-to-peak interval effectiveness, and signal quality.

In an embodiment, in the physiological parameter optimization method, providing a signal characteristic extraction unit that is configured to extract an input signal and output a signal characteristic of the input signal, extract a physiological signal and output a signal characteristic of the physiological signal when the input signal is the physiological signal, and extract a motion signal and output a signal characteristic of the motion signal when the input signal is the motion signal.

In an embodiment, before the signal characteristic of the physiological signal is determined from the physiological signal and the signal characteristic of the motion signal is determined from the motion signal, the physiological signal and the motion signal are first preprocessed in parallel.

In an embodiment, the preprocessing comprises: performing at least one of filtering, amplification, and analog-to-digital conversion on the signals.

In an embodiment, said grading the motion state during each heartbeat period of the monitored object according to the signal characteristic of the motion signal, to obtain a motion grade during each heartbeat period of the monitored object comprises: calculating a mean value or a variance of the signal characteristics of the motion signal, and grading the motion state during each heartbeat period of the monitored object through threshold segmentation, to obtain the motion grade during each heartbeat period of the monitored object.

In an embodiment, the motion grade comprises: a low motion grade for indicating that the monitored object has no motion or slight motion, a high motion grade for indicating that the monitored object has intense motion, and a medium motion grade between the low motion grade and the high motion grade.

In an embodiment, said determining an effectiveness of the physiological signal during the heartbeat period, or correcting the signal characteristic of the physiological signal during the heartbeat period when calculating a physiological parameter, or directly correcting the physiological parameter, according to the motion state during the heartbeat period comprises: determining an effectiveness of the physiological signal according to the motion grade during the heartbeat period, or correcting the signal characteristic of the physiological signal during the heartbeat period according to the motion grade during the heartbeat period when calculating the physiological parameter, or directly correcting the physiological parameter according to the motion grade during the heartbeat period.

In an embodiment, the signal characteristic comprises signal quality SQI; and said correcting the signal characteristic of the physiological signal during the heartbeat period according to the motion grade during the heartbeat period when calculating the physiological parameter comprises: correcting the signal quality SQI of the physiological signal during the heartbeat period according to the motion grade during the heartbeat period when calculating the physiological parameter.

In an embodiment, said determining an effectiveness of the physiological signal according to the motion grade during the heartbeat period comprises: when the motion grade during the heartbeat period is a low motion grade, determining that the physiological signal during the heartbeat period is effective; when the motion grade during the heartbeat period is a medium motion grade, reconfirming the effectiveness of the physiological signal during the heartbeat period; and when the motion grade during the heartbeat period is the high motion grade, determining that the physiological signal during the heartbeat period is ineffective.

In an embodiment, said correcting the signal characteristic of the physiological signal during the heartbeat period according to the motion grade during the heartbeat period when calculating the physiological parameter comprises: when the motion grade during the heartbeat period is a low motion grade the signal characteristic of the physiological signal is not modified; and when the motion grade during the heartbeat period is a medium motion grade or the high motion grade, correcting the signal characteristic of the physiological signal.

In an embodiment, said correcting the signal characteristic of the physiological signal during the heartbeat period when calculating a physiological parameter comprises: correcting weights/a weight of a time domain characteristic and/or a frequency domain characteristic of the physiological signal during the heartbeat period when calculating the physiological parameter.

In an embodiment, when determining the physiological signal is unreliable, the physiological parameter optimization method further comprises: directly calculating the physiological parameter based on the signal characteristic of the physiological signal determined from the physiological signal; and determining whether the calculated physiological parameter during the heartbeat period is effective according to the effectiveness of the physiological signal during the heartbeat period.

According to a second aspect, an embodiment provides a monitoring device as defined in claim 11, the monitoring device comprising:
an electrocardiogram sensor configured to be attached to a monitored object to acquire a physiological signal of the monitored object; the physiological signal is an ECG signal;
a motion sensor configured to be attached to the monitored object to acquire a motion signal of the monitored object; and
a processor configured to:
   determine a signal characteristic of the physiological signal from the physiological signal, and determine, whether the physiological signal is reliable according to the signal characteristic of the physiological signal; if the physiological signal is unreliable, wherein the signal characteristic of the physiological signal comprises signal quality, matching of QRS complexes and an effectiveness of QRC complexes, and said determining whether the physiological signal is reliable according to the signal characteristic of the physiological signal comprises: assigning a weight to each signal characteristic of the physiological signal acquiring a reliability score of the physiological signal through voting and scoring, and determining whether the physiological signal is reliable based on the reliability score;
   when determining the physiological signal is reliable, directly calculate the physiological parameter of the monitored object based on the signal characteristic of the physiological signal;
   when the physiological signal is unreliable, determine a signal characteristic of the motion signal from the motion signal, and determine a motion state by grading the motion comprising at least a low motion grade and a high motion grade during each heartbeat period of the monitored object during a time period in which the motion signal is acquired according to the signal characteristic of the motion signal; wherein the heartbeat period is a QRS complex interval; and
   according to the motion state during the heartbeat period, determine an effectiveness of the physiological signal during the heartbeat period, wherein the signal is determined as effective based on the low motion grade and ineffective and/or invalid at the high motion grade, or correct the signal characteristic of the physiological signal based on how the motion has been graded during the heartbeat period when calculating a physiological parameter;
   wherein the signal characteristic of the physiological signal and the motion signal is a time domain characteristic and/or a frequency domain characteristic of a signal; the time domain characteristic of the signal comprises: at least one of a peak position, a peak amplitude, a peak slope, a peak width, peak effectiveness, a peak type, a peak-to-peak interval value, peak-to-peak interval effectiveness, and signal quality.

In an embodiment, the processor comprises a signal characteristic extraction unit configured to extract an input signal and output a signal characteristic of the input signal, extract a physiological signal and output a signal characteristic of the physiological signal when the input signal is the physiological signal, and extract a motion signal and output a signal characteristic of the motion signal when the input signal is the motion signal.

In an embodiment, the monitoring device further comprises a preprocessing circuit configured to preprocess the physiological signal and the motion signal in parallel before the processor determines the signal characteristic of the physiological signal from the physiological signal and determines the signal characteristic of the motion signal from the motion signal.

In an embodiment, the preprocessing circuit comprises at least one of the following:
a filter circuit configured to filter an input signal;
an amplification circuit configured to amplify an input signal; and
an analog-to-digital conversion circuit configured to perform analog-to-digital conversion on an input signal.

In an embodiment, said grading, by the processor, the motion state during each heartbeat period of the monitored object according to the signal characteristic of the motion signal, to obtain a motion grade during each heartbeat period of the monitored object comprises: calculating a mean value and/or a variance of the signal characteristics of the motion signal, and grading the motion state during each heartbeat period of the monitored object through threshold segmentation, to obtain the motion grade during each heartbeat period of the monitored object.

In an embodiment, the motion grade comprises: a low motion grade for indicating that the monitored object has no motion or slight motion, a high motion grade for indicating that the monitored object has intense motion, and a medium motion grade between the low motion grade and the high motion grade.

In an embodiment, said determining, by the processor, determining an effectiveness of the physiological signal during the heartbeat period, or correcting the signal characteristic of the physiological signal during the heartbeat period when calculating a physiological parameter, or directly correcting the physiological parameter, according to the motion state during the heartbeat period comprises: determining an effectiveness of the physiological signal according to the motion grade during the heartbeat period, or correcting the signal characteristic of the physiological signal during the heartbeat period according to the motion grade during the heartbeat period when calculating the physiological parameter, or directly correcting the physiological parameter according to the motion grade during the heartbeat period.

In an embodiment, the signal characteristic comprises signal quality SQI; and said correcting, by the processor, the signal characteristic of the physiological signal during the heartbeat period according to the motion grade during the heartbeat period when calculating the physiological parameter comprises: correcting the signal quality SQI of the physiological signal during the heartbeat period according to the motion grade during the heartbeat period when calculating the physiological parameter.

In an embodiment, said determining, by the processor, an effectiveness of the physiological signal according to the motion grade during the heartbeat period comprises: when the motion grade during the heartbeat period is a low motion grade, determining that the physiological signal during the heartbeat period is effective; when the motion grade during the heartbeat period is a medium motion grade, reconfirming the effectiveness of the physiological signal during the heartbeat period; and when the motion grade during the heartbeat period is the high motion grade, determining that the physiological signal during the heartbeat period is ineffective.

In an embodiment, said correcting, by the processor, the signal characteristic of the physiological signal during the heartbeat period according to the motion grade during the heartbeat period when calculating the physiological parameter comprises: when the motion grade during the heartbeat period is a low motion grade the signal characteristic of the physiological signal is not modified; and when the motion grade during the heartbeat period is a medium motion grade or the high motion grade, correcting the signal characteristic of the physiological signal.

In an embodiment, said correcting, by the processor, the signal characteristic of the physiological signal during the heartbeat period when calculating a physiological parameter comprises: correcting weights/a weight of a time domain characteristic and/or a frequency domain characteristic of the physiological signal during the heartbeat period when calculating the physiological parameter.

In an embodiment, when determining the physiological signal is unreliable, the processor further directly calculates the physiological parameter based on the signal characteristic of the physiological signal determined from the physiological signal; and determines whether the calculated physiological parameter during the heartbeat period is effective according to the effectiveness of the physiological signal during the heartbeat period.

In an embodiment, the monitoring device further comprises a housing, wherein the processor is arranged in the housing and is connected to the physiological sensor and the motion sensor in a wired or wireless manner.

In an embodiment, the motion sensor comprises an acceleration sensor, an angular velocity sensor, or a gravity sensor.

In an embodiment, the monitoring device is a mobile monitoring device.

According to a third aspect, an embodiment provides a computer-readable storage medium comprising a program, wherein the program can be executed by a processor to implement any one of the above methods.

According to the method for optimizing physiological parameter using a motion sensor, the monitoring device, and the computer-readable storage medium in the above embodiments, a physiological signal and a motion signal of a patient are synchronously acquired, a signal characteristic of the physiological signal is determined from the physiological signal, and whether the physiological signal is reliable is determined according to the signal characteristic of the physiological signal; when determining the physiological signal is unreliable, a signal characteristic of the motion signal is determined from the motion signal, and determining a motion state during each heartbeat period of the monitored object according to the signal characteristic of the motion signal; and an effectiveness of the physiological signal during the heartbeat period is determined according to the motion state during the heartbeat period, or the signal characteristic of the physiological signal during the heartbeat period is corrected when a physiological parameter is calculated, thereby improving accuracy of the physiological signal and the physiological parameter.

### Brief Description of the Drawings

FIG. 1 is a system framework diagram of a parameter processing module in a multi-parameter monitor;
FIG. 2 is a system framework diagram of a parameter processing module in a single-parameter monitor;
FIG. 3 is a schematic structural diagram of a monitor networking system used in a hospital;
FIG. 4 is a flowchart of a method for optimizing physiological parameter using a motion sensor according to an embodiment;
FIG. 5 is a flowchart of a method for optimizing physiological parameter using a motion sensor according to another embodiment;
FIG. 6 is a schematic diagram of correcting a signal characteristic of a physiological signal during a heartbeat period according to a motion state during the heartbeat period when calculating a physiological parameter according to an embodiment;
FIG. 7 is a schematic structural diagram of a monitoring device according to an embodiment;
FIG. 8 is a schematic structural diagram of a monitoring device according to another embodiment;
FIG. 9 is a schematic structural diagram of a monitoring device according to still another embodiment; and
FIG. 10 is a schematic structural diagram of a monitoring device according to yet another embodiment.

### Detailed Description of Embodiments

The disclosure will be further described in detail below through specific implementations in conjunction with the accompanying drawings. Associated similar element reference numerals are used for similar elements in different implementations. In the following implementations, many details are described such that the present application can be better understood. However, it would have been effortlessly appreciated by a person skilled in the art that some of the features could be omitted or could be substituted by other elements, materials, and methods in different cases. In certain cases, some operations involved in the present application are not displayed or described in the specification, which is to prevent a core part of the present application from being obscured by too much description. Moreover, for a person skilled in the art, the detailed description of the involved operations is not necessary, and the involved operations can be thoroughly understood according to the description in the specification and the general technical knowledge in the art.

In addition, the characteristics, operations, or features described in the specification can be combined in any appropriate manner to form various implementations. Moreover, the steps or actions in the method description can also be exchanged or adjusted in order in a way that would have been obvious to a person skilled in the art. Therefore, the various orders in the specification and the accompanying drawings are merely for the purpose of clear description of a certain embodiment and are not meant to be a necessary order unless otherwise stated that a certain order must be followed.

The serial numbers themselves for the components herein, for example, "first" and "second", are merely used to distinguish the described objects, and do not have any sequential or technical meaning. Moreover, as used in the present application, "connection" or "coupling", unless otherwise specified, comprises both direct and indirect connections (couplings).

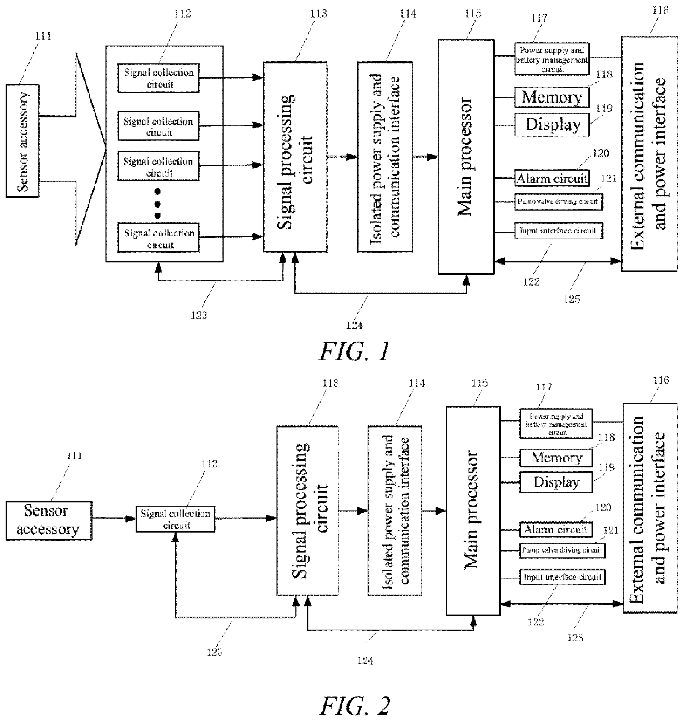

As shown in FIG. 1, a system framework diagram of a parameter processing module in a multi-parameter monitor is provided. The multi-parameter monitor has an independent housing, and a sensor interface area is arranged on a housing panel. A plurality of sensor interfaces are integrated in the sensor interface area and configured to be connected to various external physiological parameter sensor accessories 111. The housing panel further comprises a small IXD display area, a display 119, an input interface circuit 122, an alarm circuit 120 (such as an LED alarm area), and the like. The parameter processing module has an external communication and power interface for communicating with a host and obtaining power from the host. The parameter processing module further supports an externally inserted parameter module. The parameter module may be inserted to form a plug-in monitor host as a part of the monitor, or may be connected to the host via a cable, where the externally inserted parameter module serves as an external accessory of the monitor.

An internal circuit of the parameter processing module is placed in the housing. As shown in FIG. 1, the internal circuit comprises signal collection circuits 112 corresponding to at least two physiological parameters, a front-end signal processing circuit 113, and a main processor 115. The signal collection circuit 112 may be selected from an electrocardiogram circuit, a respiration circuit, a body temperature circuit, a blood oxygen circuit, a non-invasive blood pressure circuit, an invasive blood pressure circuit, and the like. These signal collection circuits 112 are respectively electrically connected to corresponding sensor interfaces, so as to be electrically connected to sensor accessories 111 corresponding to different physiological parameters. An output end of the signal collection circuit is coupled to a front-end signal processor, a communication port of the front-end signal processor is coupled to the main processor, and the main processor is electrically connected to the external communication and power interface. Various physiological parameter measurement circuits can use common circuits in the prior art. The front-end signal processing circuit completes sampling and analog-to-digital conversion of an output signal of the signal collection circuit, and outputs a control signal to control a measurement process of a physiological signal. These parameters comprise but are not limited to: parameters such as electrocardiogram, respiration, body temperature, blood oxygen, non-invasive blood pressure, and invasive blood pressure. The front-end signal processing circuit may be implemented by using a single-chip microcomputer or other semiconductor devices, for example, by using a single-chip microcomputer, an ASIC, or an FPGA. The front-end signal processing circuit may also be powered by an isolated power supply, and data obtained through sampling may be sent to the main processor through an isolated communication interface after being simply processed and packaged. For example, the front-end signal processing circuit 113 may be coupled to the main processor 115 through an isolated power supply and communication interface 114. The reason why the front-end signal processing circuit is powered by the isolated power supply is that the DC/DC power supply isolated by a transformer plays a role in isolating a patient from a power supply apparatus, with the main purposes of: 1. isolating the patient, and enabling an application part to be floating by means of an isolation transformer, so that a leakage current of the patient is small enough; and 2. preventing the voltage or energy during defibrillation or electrotome application from affecting a board card and a device of an intermediate circuit such as a main control board (guaranteed by a creepage distance and electrical clearance). Certainly, the front-end signal processor circuit 113 may also be connected to the main processor 115 by means of a cable 124. The main processor completes calculation of the physiological parameter, and sends a calculation result and waveforms of the parameter to the host (such as a host with a display, a PC, and a central station) through the external communication and power interface 116. The main processor 115 may also be connected to the external communication and power interface 116 by means of a cable 125, and the external communication and power interface 116 may be one of local area network interfaces composed of the Ethernet, a token ring, a token bus, and a fiber distributed data interface (FDDI) as the backbone network of these three networks, or a combination thereof, or may be one of wireless interfaces such as an infrared interface, a Bluetooth interface, a Wi-Fi interface, and a WMTS communication interface, or a combination thereof, or may be one of wired data connection interfaces such as an RS232 interface and a USB interface, or a combination thereof. The external communication and power interface 116 may also be one of a wireless data transmission interface and a wired data transmission interface or a combination thereof. The host may be any computer apparatus such as a host of a monitor, an electrocardiograph, an ultrasonic diagnostic apparatus, and a computer, and after being installed with matching software, the host can form a monitoring apparatus. The host may further be a communication apparatus such as a mobile phone, and the parameter processing module sends, by using a Bluetooth interface, data to the mobile phone supporting Bluetooth communication, so as to implement remote transmission of the data. After completing the calculation of the physiological parameter, the main processor 115 may further determine whether the physiological parameter is abnormal, and if yes, may give an alarm by means of the alarm circuit 120. In addition, a power supply and battery management circuit 117 in the figure is configured to manage and process power supply of the monitor, and a memory 118 may store intermediate and final data of the monitor, and store a program instruction or code executed by the main processor 115 and the like. If the monitor has a function of blood pressure measurement, the monitor may further comprise a pump valve driving circuit 121. The pump valve driving circuit 121 is configured to perform inflation or deflation operations under the control of the main processor 115. If the monitor does not have the function of blood pressure measurement, the pump valve driving circuit 121 may be eliminated.

As shown in FIG. 2, a processing system architecture of a monitor with a single physiological parameter is provided. For the same content, reference may be made to the above content.

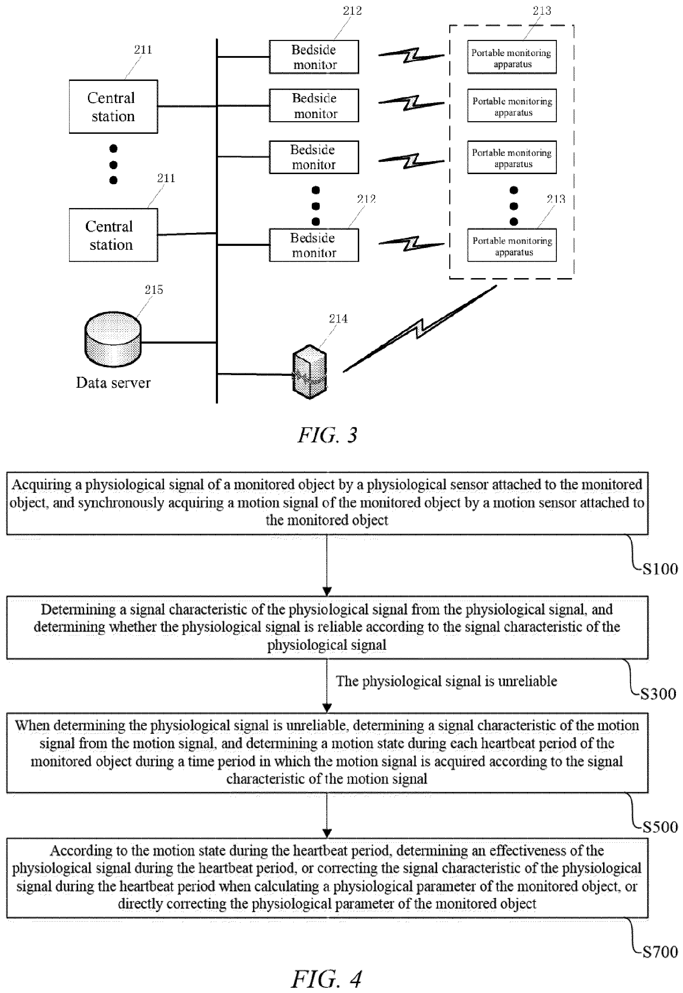

As shown in FIG. 3, a monitor networking system used in a hospital is provided. By using the system, data of the monitor may be saved as a whole to centrally manage patient information and nursing information that are stored in association, which facilitates storage of historical data and alarming in association. In the system shown in FIG. 3, a bedside monitor 212 may be provided for each hospital bed. The bedside monitor 212 may be the above multi-parameter monitor or a plug-in monitor. In addition, each bedside monitor 212 may further be paired with one portable monitoring apparatus 213 for transmission. The portable monitoring apparatus 213 provides a simple and portable parameter processing module, and the simple and portable parameter processing module may be worn on the body of a patient to perform mobile monitoring for the patient. After the portable monitoring apparatus 213 and the bedside monitor 212 perform wired or wireless communication, physiological data generated through mobile monitoring may be transmitted to the bedside monitor 212 for display, or transmitted, by using the bedside monitor 212, to a central station 211 for a doctor or a nurse to check, or transmitted to a data server 215 for storage by using the bedside monitor 212. In addition, the portable monitoring apparatus 213 may further directly transmit, by using a wireless network node 214 arranged in the hospital, the physiological data generated through mobile monitoring to the central station 211 for storage and display, or transmit, by using the wireless network node 214 arranged in the hospital, the physiological data generated through mobile monitoring to the data server 215 for storage. It can be seen that the data corresponding to the physiological parameter displayed on the bedside monitor 212 may originate from a sensor accessory directly connected to the monitor, or from the portable monitoring apparatus 213, or from the data server. The portable monitoring apparatus 213 may be connected to the sensor accessory 111 in a wired and/or wireless manner, and comprise a part or all of the circuits of the above parameter processing module. For example, isolation measures for isolating patients may not be provided in the portable monitoring apparatus 213 but provided outside the portable monitoring apparatus 213, for example, provided on the sensor accessory 111. The portable monitoring apparatus 213 may be equipped with a display screen for displaying a parameter calculation result and/or alarm prompt information. For example, the portable monitoring apparatus 213 may be a wireless sensor patch attached to the body, or a transfer monitor, or a telemetry apparatus.

Referring to FIG. 4, an embodiment provides a method for optimizing physiological parameter using a motion sensor (hereinafter referred to as a physiological parameter optimization method). The method may be applied to the monitor mentioned above, and comprises steps S100 to S700, which will be described in detail below.

Step S100, acquiring a physiological signal of a monitored object by a physiological sensor attached to the monitored object, and synchronously acquiring a motion signal of the monitored object by a motion sensor attached to the monitored object.

In an embodiment, the motion sensor is a sensor configured to sense the motion of the monitored object. For example, the motion sensor may comprise at least one of a speed sensor, an acceleration sensor, an angular velocity sensor (such as a gyroscope), or a gravity sensor, and corresponding motion signals are respectively a speed signal (which may be a velocity vector with a direction or a rate without a direction), an acceleration signal, an angular velocity signal, and a gravitational acceleration signal. Certainly, the motion signal may also be a duration of the motion. The motion sensor is attached to the monitored object, and may be directly attached to the body or clothes of the monitored object. For example, the motion sensor may be placed on an electrode lead wire, or placed on a device adjacent to the electrode/lead wire, or integrated with the electrode. When the monitor is a mobile monitor, the motion sensor may be arranged on a circuit board in the monitor.

Step S300, determining a signal characteristic of the physiological signal from the physiological signal, and determining whether the physiological signal is reliable according to the signal characteristic of the physiological signal.

In an embodiment, said determining, according to the signal characteristic of the physiological signal, whether the physiological signal is reliable may comprise: assigning a weight to each signal characteristic, acquiring a reliability score through voting and scoring, and determining whether the physiological signal is reliable based on the reliability score. For example, by means of a machine learning algorithm, a weight is assigned to each signal characteristic of the physiological signal, the reliability score of the physiological signal is acquired by voting and scoring on these signal characteristics of the physiological signal, and then whether the physiological signal is reliable is determined based on the reliability score. For example, a reliability threshold is set. If the reliability score is greater than the reliability threshold, it is determined that the physiological signal is reliable; otherwise, it is determined that the physiological signal is unreliable. For example, it is assumed that the physiological signal has three signal characteristics, that is, signal quality SQI, matching of QRS complexes, and effectiveness of QRS complexes, and weights may be respectively set for the three signal characteristics. For example, a weight of the signal quality SQI is 50%, a weight of the matching of QRS complexes is 30%, and a weight of the effectiveness of QRS complexes is 20%. Then voting and scoring are performed on the three signal characteristics. For example, higher signal quality SQI leads to a higher score of the signal quality. For example, the full score is 10 points, and higher signal quality SQI leads to a score of the signal quality being closer to 10 points. Similarly, better matching of QRS complexes leads to a higher score of the matching of QRS complexes, and better effectiveness of QRS complexes leads to a higher score of the effectiveness of QRS complexes. Certainly, simple division may be performed for the matching of QRS complexes and the effectiveness of QRS complexes, where if the matching of QRS complexes is good, a score is obtained, and if the matching of QRS complexes is not good, another score is obtained, or if a QRS complex is effective, a score is obtained, and if the QRS complex is ineffective, another score is obtained. Then, scores of the three signal characteristics are multiplied by the weights thereof and products are added to obtain the reliability score of the physiological signal, and finally whether the physiological signal is reliable is determined based on the reliability score. Certainly, a plurality of thresholds about reliability may also be set, and then reliability grade classification is performed on the physiological signal according to the reliability score of the physiological signal, and finally the physiological signal is normalized to be reliable or unreliable.

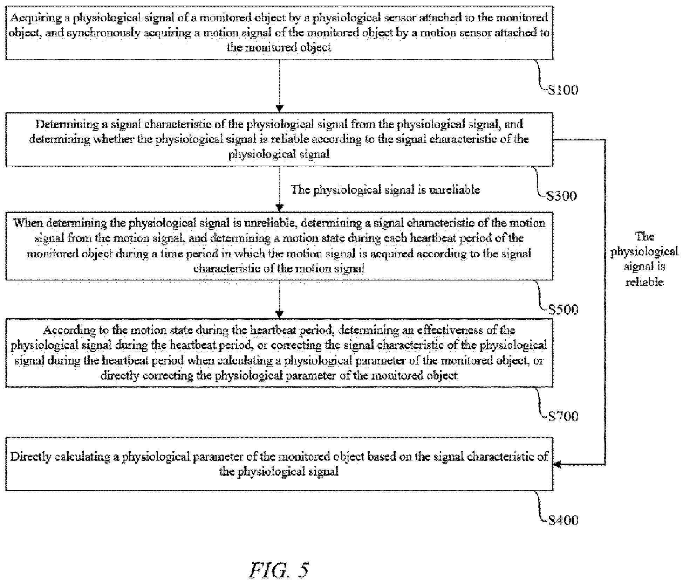

Referring to FIG. 5, in an embodiment, the physiological parameter optimization method of the disclosure may further comprise step S400, when determining that the physiological signal is reliable, directly calculating a physiological parameter based on the signal characteristic of the physiological signal.

Step S500, when determining the physiological signal is unreliable, determining a signal characteristic of the motion signal from the motion signal, and determining a motion state during each heartbeat period of the monitored object according to the signal characteristic of the motion signal. The heartbeat period represents a duration of a heartbeat, for example, the heartbeat period may be understood as a QRS complex interval.

In an embodiment, said determining a motion state during each heartbeat period of the monitored object according to the signal characteristic of the motion signal may comprise: grading the motion state during each heartbeat period of the monitored object according to the signal characteristic of the motion signal, to obtain a motion grade during each heartbeat period of the monitored object. For example, a mean value and/or a variance of signal characteristics of the motion signal may be calculated, and the motion state during each heartbeat period of the monitored object may be graded through threshold segmentation, to obtain the motion grade during each heartbeat period of the monitored object. For example, motion signals are graded by setting different segmentation thresholds.

In an embodiment, the motion grade may comprise three grades, for example, a low motion grade for indicating that the monitored object has no motion or slight motion, a high motion grade for indicating that the monitored object has intense motion, and a medium motion grade between the low motion grade and the high motion grade. In other embodiments, the motion grade may also be divided into other numbers of grades, for example, two grades (for example, only the low motion grade and the high motion grade are retained) and four grades.

Step S300 involves the signal characteristic of the physiological signal, and step S500 involves the signal characteristic of the motion signal. In an embodiment, the signal characteristic may comprise a time domain characteristic and/or a frequency domain characteristic of a signal. The time domain characteristic of the signal may comprise: at least one of a peak position, a peak amplitude, a peak slope, a peak width, peak effectiveness, a peak type, a peak-to-peak interval value, peak-to-peak interval effectiveness, and signal quality SQI. There are many specific implementations. For example, the time domain signal is used as an example, where peak searching may be performed on the signal, a peak amplitude, a peak slope, and a peak width may be calculated, and signal quality SQI and other time domain characteristics such as peak effectiveness, a peak type, a peak-to-peak interval value, and interval effectiveness may be further calculated. For example, the frequency domain characteristic is used as an example, where Fourier Transform may be performed on the signal to obtain a frequency characteristic of the signal, and then a high-frequency signal and a low-frequency signal are processed to obtain a required spectrum characteristic. In some embodiments, in order to better extract the signal characteristic of the signal, the signal may further be filtered and denoised first, for example, power frequency interference, baseline drift, and high frequency noise interference of the signal are filtered out.

It can be seen that, according to the method of the disclosure, the signal characteristic of the physiological signal and the signal characteristic of the motion signal are introduced in a physiological parameter optimization process, and the purpose of physiological parameter optimization is finally achieved with the signal characteristics of the two types of signals. However, in a specific implementation process, a signal characteristic extraction unit may be provided and configured to: extract extract an input signal and output a signal characteristic of the input signal, extract a physiological signal and output a signal characteristic of the physiological signal when the input signal is the physiological signal, and extract a motion signal and output a signal characteristic of the motion signal when the input signal is the motion signal. The signal characteristic of the physiological signal and the signal characteristic of the motion signal are extracted by using a same hardware structure, so that hardware unlocking can be effectively reduced, and costs can be reduced.

In an embodiment, in order to preferably obtain the signal characteristic of the signal, before the signal characteristic of the physiological signal is determined from the physiological signal and the signal characteristic of the motion signal is determined from the motion signal, the physiological signal and the motion signal are first preprocessed in parallel, for example, at least one of filtering, amplification, and analog-to-digital conversion may be performed on the signal. For example, the physiological sensor and the motion sensor respectively acquire the physiological signal and the motion signal. The two types of signals are first filtered to remove noise and then amplified to enlarge signal amplitudes. Finally, the physiological signal and the motion signal are converted from analog signals into digital signals through analog-to-digital conversion.

Step S700, according to the motion state during the heartbeat period, determining an effectiveness of the physiological signal during the heartbeat period, or correcting the signal characteristic of the physiological signal during the heartbeat period when calculating a physiological parameter, or directly correcting the physiological parameter.

In an embodiment, said determining an effectiveness of the physiological signal during the heartbeat period according to the motion state during the heartbeat period may comprise: determining an effectiveness of the physiological signal according to the motion grade during the heartbeat period. For example, when the motion grade during the heartbeat period is the low motion grade, it may be determined that the physiological signal during the heartbeat period is effective; when the motion grade during the heartbeat period is the medium motion grade, the effectiveness of the physiological signal during the heartbeat period is reconfirmed; and when the motion grade during the heartbeat period is the high motion grade, it is determined that the physiological signal during the heartbeat period is ineffective. The reconfirmation of the effectiveness of the physiological signal during the heartbeat period may be further confirming the effectiveness of these physiological signals in combination with the motion characteristic of the motion signal, or reconfirming the effectiveness of these physiological signals after the physiological signals are corrected.

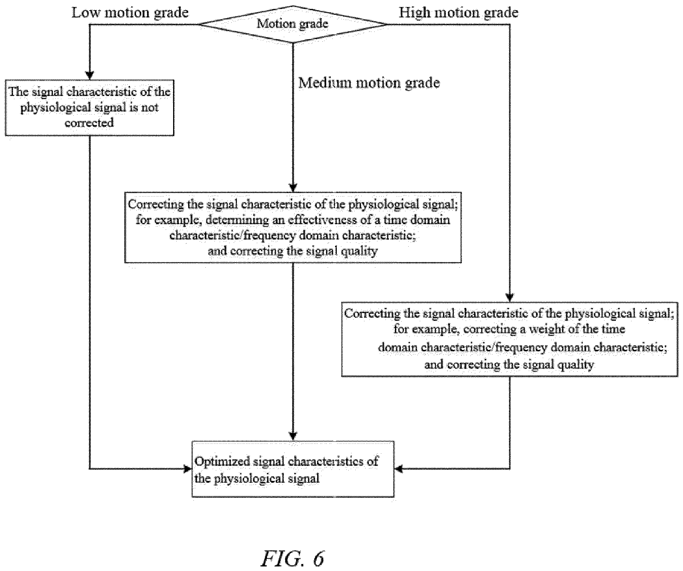

Referring to FIG. 6, in an embodiment, said correcting the signal characteristic of the physiological signal during the heartbeat period according to the motion state during the heartbeat period when calculating a physiological parameter may comprise: correcting the signal characteristic of the physiological signal during the heartbeat period according to the motion grade during the heartbeat period when calculating the physiological parameter. For example, when the motion grade during the heartbeat period is the low motion grade, the signal characteristic of the physiological signal is not corrected; and when the motion grade during the heartbeat period is the medium motion grade or the high motion grade, the signal characteristic of the physiological signal is corrected. For example, when the motion grade during the heartbeat period is the medium motion grade, effectiveness of a time domain characteristic and/or a frequency domain characteristic of the physiological signal is determined, and a grade/threshold of SQI may also be corrected; and when the motion grade during the heartbeat period is the high motion grade, weights/a weight (which may be set to 0) of the time domain characteristic and/or the frequency domain characteristic of the physiological signal are/is corrected, and the grade/threshold of the SQI may also be corrected. Alternatively, for example, signal quality SQI of the physiological signal during the heartbeat period may be corrected according to the motion grade during the heartbeat period when calculating the physiological parameter. The signal characteristic may comprise the signal quality SQI, for example, a grade or threshold of the signal quality SQI may be corrected. For example, when the signal characteristic of the physiological signal is determined from the physiological signal, signal quality SQI is obtained, and then a value of the signal quality SQI may be corrected according to the motion grade during the heartbeat period. For another example, a corresponding SQI value may be pre-stored for each motion grade, and when the signal characteristic of the physiological signal is determined from the physiological signal, signal quality SQI is obtained, and the obtained signal quality SQI may be directly replaced according to an SQI value corresponding to the motion grade during the heartbeat period. Alternatively, for example, weights/a weight of a time domain characteristic and/or a frequency domain characteristic of the physiological signal during the heartbeat period may be corrected when the physiological parameter is calculated. The signal characteristic may comprise a time domain characteristic and/or a frequency domain characteristic of a signal.

However, in another embodiment, when the motion grade during the heartbeat period is the low motion grade, it may be determined that the physiological signal during the heartbeat period is effective (therefore, the physiological parameter generated according to the physiological signal is also effective). When the motion grade during the heartbeat period is the medium motion grade, the physiological signal (such as a QRS complex interval, an SQI value, and a QRS complex type) is processed, for example, correction processing is performed. When the motion grade during the heartbeat period is the high motion grade, the physiological parameter (a heart rate value, arrhythmia, a blood oxygen value, and blood oxygen saturation) generated according to the physiological signal is directly processed, for example, correction processing is performed. In this embodiment, it may be directly determined, according to the motion grade, that the physiological signal is effective and therefore the physiological parameter is also effective, the physiological signal may be corrected, or the physiological parameter may be corrected directly.

In an embodiment, the physiological parameter optimization method of the disclosure may further comprise a step. If it is determined that the physiological signal is unreliable, the method further comprises: directly calculating the physiological parameter based on the signal characteristic of the physiological signal determined from the physiological signal; and determining whether the calculated physiological parameter during the heartbeat period is effective according to the effectiveness of the physiological signal during the heartbeat period. For example, after the physiological parameter is directly calculated based on the physiological signal characteristic determined from the physiological signal, if the motion grade is the low motion grade, it is determined that the directly calculated physiological parameter is effective, if the motion grade is the medium motion grade, it is determined that the directly calculated physiological parameter is in doubt, and if the motion grade is the high motion grade, it is determined that the directly calculated physiological parameter is ineffective.

The above are some content of the physiological parameter optimization method of the disclosure. According to the method, a physiological signal of a monitored object is acquired, and a signal characteristic of the physiological signal is determined based on the physiological signal of the monitored object, and then reliability of the physiological signal is determined based on the signal characteristic of the physiological signal. In addition, a motion signal of the monitored object is acquired, and a signal characteristic of the motion signal is determined based on the motion signal of the monitored object, so as to further determine a motion state during each heartbeat period of the monitored object. When it is determined that the physiological signal is unreliable, optimization is performed according to the motion state during the heartbeat period, thereby improving accuracy of a physiological parameter and reducing false alarms.

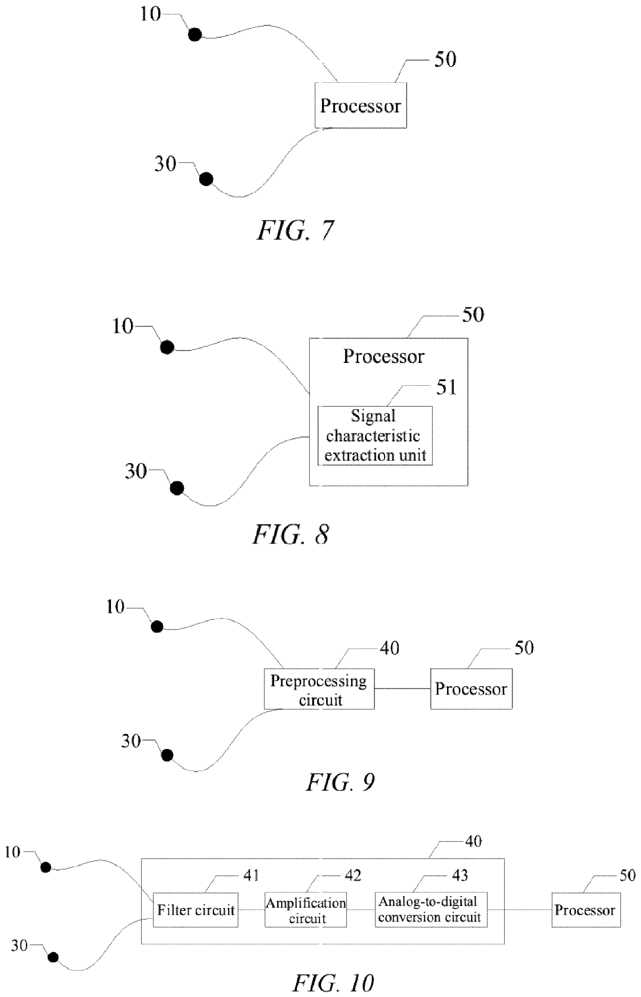

Referring to FIG. 7, an embodiment of the disclosure further discloses a monitoring device or a monitor, and the monitoring device or the monitor may comprise a physiological sensor 10, a motion sensor 30, and a processor 50. In an embodiment, the monitoring device further comprises a housing, wherein the processor 50 is arranged in the housing and is connected to the physiological sensor 10 and the motion sensor 30 in a wired or wireless manner. Detailed description is given below.

The physiological sensor 10 is configured to be attached to a monitored object to acquire a physiological signal of the monitored object.

The motion sensor 30 is configured to be attached to the monitored object to acquire a motion signal of the monitored object. In an embodiment, the motion sensor is a sensor configured to sense the motion of the monitored object. For example, the motion sensor may comprise at least one of a speed sensor, an acceleration sensor, an angular velocity sensor (such as a gyroscope), or a gravity sensor, and corresponding motion signals are respectively a speed signal (which may be a velocity vector with a direction or a rate without a direction), an acceleration signal, an angular velocity signal, and a gravitational acceleration signal. Certainly, the motion signal may also be a duration of the motion. The motion sensor is attached to the monitored object, and may be directly attached to the body or clothes of the monitored object, or may be attached to the housing of the monitoring device. For example, the motion sensor may be placed on an electrode lead wire, or placed on a device adjacent to the electrode/lead wire, or integrated with the electrode. When the monitor is a mobile monitor, the motion sensor may be arranged on a circuit board in the monitor.

The processor 50 is configured to: determine a signal characteristic of the physiological signal from the physiological signal, and determine whether the physiological signal is reliable according to the signal characteristic of the physiological signal; when the physiological signal is unreliable, determine a signal characteristic of the motion signal from the motion signal, and determine a motion state during each heartbeat period of the monitored object according to the signal characteristic of the motion signal; and according to the motion state during the heartbeat period, determine an effectiveness of the physiological signal during the heartbeat period, or correct the signal characteristic of the physiological signal during the heartbeat period when calculating a physiological parameter, or directly correct the physiological parameter. In an embodiment, the processor 50 may acquire the physiological signal of the monitored object by the physiological sensor attached to the monitored object, and synchronously acquire the motion signal of the monitored object by the motion sensor attached to the monitored object.

From the description above, the processor 50 first determines reliability of the physiological signal; and performs optimization with the motion signal when determining that the physiological signal is unreliable, which is to be described in detail below.

When determining the reliability of the physiological signal, the processor 50 may first determine the signal characteristic of the physiological signal from the physiological signal, and determine whether the physiological signal is reliable according to the signal characteristic of the physiological signal. For example, the processor 50 may assigning a weight to each signal characteristic of the physiological signal acquiring a reliability score through voting and scoring, and determining whether the physiological signal is reliable based on the reliability score. Specifically, for example, by means of a machine learning algorithm, a weight is assigned to each signal characteristic of the physiological signal, the reliability score of the physiological signal is acquired by voting and scoring on these signal characteristics of the physiological signal, and then whether the physiological signal is reliable is determined based on the reliability score. For example, a reliability threshold is set. If the reliability score is greater than the reliability threshold, it is determined that the physiological signal is reliable; otherwise, it is determined that the physiological signal is unreliable. For example, it is assumed that the physiological signal has three signal characteristics, that is, signal quality SQI, matching of QRS complexes, and effectiveness of QRS complexes, and weights may be respectively set for the three signal characteristics. For example, a weight of the signal quality SQI is 50%, a weight of the matching of QRS complexes is 30%, and a weight of the effectiveness of QRS complexes is 20%. Then voting and scoring are performed on the three signal characteristics. For example, higher signal quality SQI leads to a higher score of the signal quality. For example, the full score is 10 points, and higher signal quality SQI leads to a score of the signal quality being closer to 10 points. Similarly, better matching of QRS complexes leads to a higher score of the matching of QRS complexes, and better effectiveness of QRS complexes leads to a higher score of the effectiveness of QRS complexes. Certainly, simple division may be performed for the matching of QRS complexes and the effectiveness of QRS complexes, where if the matching of QRS complexes is good, a score is obtained, and if the matching of QRS complexes is not good, another score is obtained, or if a QRS complex is effective, a score is obtained, and if the QRS complex is ineffective, another score is obtained. Then, scores of the three signal characteristics are multiplied by the weights thereof and products are added to obtain the reliability score of the physiological signal, and finally whether the physiological signal is reliable is determined based on the reliability score. Certainly, a plurality of thresholds about reliability may also be set, and then reliability grade classification is performed on the physiological signal according to the reliability score of the physiological signal, and finally the physiological signal is normalized to be reliable or unreliable.

When determining the physiological signal is reliable, the processor 50 directly calculates the physiological parameter based on the signal characteristic of the physiological signal; and when determining the physiological signal is unreliable, the processor 50 performs subsequent optimization.

When determining the physiological signal is unreliable, the processor 50 determines the signal characteristic of the motion signal from the motion signal, and determines the motion state during each heartbeat period of the monitored object according to the signal characteristic of the motion signal. For example, in an embodiment, the processor 50 may grade the motion state during each heartbeat period of the monitored object according to the signal characteristic of the motion signal, to obtain a motion grade during each heartbeat period of the monitored object. For example, the processor 50 may calculate a mean value and/or a variance of signal characteristics of the motion signal, and grade the motion state during each heartbeat period of the monitored object through threshold segmentation, to obtain the motion grade during each heartbeat period of the monitored object. For example, the processor 50 grades motion signals by setting different segmentation thresholds. In an embodiment, the motion grade may comprise three grades, for example, a low motion grade for indicating that the monitored object has no motion or slight motion, a high motion grade for indicating that the monitored object has intense motion, and a medium motion grade between the low motion grade and the high motion grade. In other embodiments, the motion grade may also be divided into other numbers of grades, for example, two grades (for example, only the low motion grade and the high motion grade are retained) and four grades.

According to the motion state during the heartbeat period, the processor 50 then determines the effectiveness of the physiological signal during the heartbeat period, or corrects the signal characteristic of the physiological signal during the heartbeat period when calculating the physiological parameter, or directly corrects the physiological parameter.

In an embodiment, said determining, by the processor 50, an effectiveness of the physiological signal during the heartbeat period according to the motion state during the heartbeat period may comprise: determining an effectiveness of the physiological signal according to the motion grade during the heartbeat period. For example, when the motion grade during the heartbeat period is the low motion grade, the processor 50 may determine that the physiological signal during the heartbeat period is effective; when the motion grade during the heartbeat period is the medium motion grade, the processor reconfirms the effectiveness of the physiological signal during the heartbeat period; and when the motion grade during the heartbeat period is the high motion grade, the processor determines that the physiological signal during the heartbeat period is ineffective. The reconfirmation of the effectiveness of the physiological signal during the heartbeat period may be further confirming the effectiveness of these physiological signals in combination with the motion characteristic of the motion signal, or reconfirming the effectiveness of these physiological signals after the physiological signals are corrected.

In an embodiment, said correcting, by the processor 50, the signal characteristic of the physiological signal according to the motion state during the heartbeat period during the heartbeat period when calculating a physiological parameter may comprise: correcting the signal characteristic of the physiological signal during the heartbeat period according to the motion grade during the heartbeat period when calculating the physiological parameter. For example, when the motion grade during the heartbeat period is the low motion grade, the processor 50 does not correct the signal characteristic of the physiological signal; and when the motion grade during the heartbeat period is the medium motion grade or the high motion grade, the processor corrects the signal characteristic of the physiological signal. For example, when the motion grade during the heartbeat period is the medium motion grade, effectiveness of a time domain characteristic and/or a frequency domain characteristic of the physiological signal is determined, and a grade/threshold of SQI may also be corrected; and when the motion grade during the heartbeat period is the high motion grade, weights/a weight (which may be set to 0) of the time domain characteristic and/or the frequency domain characteristic of the physiological signal are/is corrected, and the grade/threshold of the SQI may also be corrected. Alternatively, for example, the processor 50 may correct signal quality SQI of the physiological signal during the heartbeat period according to the motion grade during the heartbeat period when calculating the physiological parameter. The signal characteristic may comprise the signal quality SQI, for example, a grade or threshold of the signal quality SQI may be corrected. For example, when the signal characteristic of the physiological signal is determined from the physiological signal, signal quality SQI is obtained, and then a value of the signal quality SQI may be corrected according to the motion grade during the heartbeat period. For another example, a corresponding SQI value may be pre-stored for each motion grade, and when the signal characteristic of the physiological signal is determined from the physiological signal, signal quality SQI is obtained, and the obtained signal quality SQI may be directly replaced according to an SQI value corresponding to the motion grade during the heartbeat period. Alternatively, for example, the processor 50 may correct weights/a weight of a time domain characteristic and/or a frequency domain characteristic of the physiological signal during the heartbeat period when calculating the physiological parameter. The signal characteristic may comprise a time domain characteristic and/or a frequency domain characteristic of a signal.

However, in another embodiment, when the motion grade during the heartbeat period is the low motion grade, the processor 50 may determine that the physiological signal during the heartbeat period is effective (therefore, the physiological parameter generated according to the physiological signal is also effective). When the motion grade during the heartbeat period is the medium motion grade, the physiological signal (such as a QRS complex interval, an SQI value, and a QRS complex type) is processed, for example, correction processing is performed. When the motion grade during the heartbeat period is the high motion grade, the physiological parameter (a heart rate value, arrhythmia, a blood oxygen value, and blood oxygen saturation) generated according to the physiological signal is directly processed, for example, correction processing is performed. In this embodiment, it may be directly determined, according to the motion grade, that the physiological signal is effective and therefore the physiological parameter is also effective, the physiological signal may be corrected, or the physiological parameter may be corrected directly.

In an embodiment, when determining the physiological signal is unreliable, the processor 50 may further directly calculate the physiological parameter based on the signal characteristic of the physiological signal determined from the physiological signal; and determine whether the calculated physiological parameter during the heartbeat period is effective according to the effectiveness of the physiological signal during the heartbeat period. For example, after directly calculating the physiological parameter based on the physiological signal characteristic determined from the physiological signal, if the motion grade is the low motion grade, the processor 50 determines that the directly calculated physiological parameter is effective, if the motion grade is the medium motion grade, the processor determines that the directly calculated physiological parameter is in doubt, and if the motion grade is the high motion grade, the processor determines that the directly calculated physiological parameter is ineffective.

It can be seen from the above description of the processor 50 that the processor 50 is involved in determining the signal characteristic of the physiological signal and the signal characteristic of the motion signal. In an embodiment, the signal characteristic may comprise a time domain characteristic and/or a frequency domain characteristic of a signal. The time domain characteristic of the signal may comprise: at least one of a peak position, a peak amplitude, a peak slope, a peak width, peak effectiveness, a peak type, a peak-to-peak interval value, peak-to-peak interval effectiveness, and signal quality SQI. There are many specific ways for the processor 50 to determine the signal characteristic of the signal. For example, the time domain signal is used as an example, where the processor 50 may perform peak searching on the signal, calculate a peak amplitude, a peak slope, and a peak width, and further calculate signal quality SQI and other time domain characteristics such as peak effectiveness, a peak type, a peak-to-peak interval value, and interval effectiveness. For example, the frequency domain characteristic is used as an example, where the processor 50 may perform Fourier Transform on the signal to obtain a frequency characteristic of the signal, and then process a high-frequency signal and a low-frequency signal to obtain a required spectrum characteristic. In order to reduce hardware unlocking and costs, in an embodiment, referring to FIG. 8, the processor 50 may comprise a signal characteristic extraction unit 51 configured to: extract an input signal and output a signal characteristic of the input signal, extract a physiological signal and output a signal characteristic of the physiological signal when the input signal is the physiological signal, and extract a motion signal and output a signal characteristic of the motion signal when the input signal is the motion signal. Referring to FIG. 9, in an embodiment, in order to better obtain the signal characteristic of the signal, the monitoring device of the disclosure may further comprise a preprocessing circuit 40 configured to first preprocess the physiological signal and the motion signal in parallel before the processor determines the signal characteristic of the physiological signal from the physiological signal and determines the signal characteristic of the motion signal from the motion signal. For example, referring to FIG. 10, the preprocessing circuit 40 may comprise at least one of a filter circuit 41, an amplification circuit 42, and an analog-to-digital conversion circuit 43. The filter circuit 41 is configured to filter an input signal, the amplification circuit 42 is configured to amplify the input signal, and the analog-to-digital conversion circuit 43 is configured to perform analog-to-digital conversion on the input signal. The filter circuit 41 may be used as a front stage of the preprocessing circuit 40, the amplification circuit 42 may be used as an intermediate stage of the preprocessing circuit 40, and the analog-to-digital conversion circuit 43 may be used as a rear stage of the preprocessing circuit. For example, the preprocessing circuit 40 first filters the signal by means of the filter circuit 41 to remove noise, then amplifies the filtered signal by means of the amplification circuit 42 to enlarge a signal amplitude, and finally performs analog-to-digital conversion on the amplified signal by means of the analog-to-digital conversion circuit 43 to convert the signal from an analog signal to a digital signal.

It should be noted that for some structures and components of the monitoring device disclosed in FIG. 7 to FIG. 10, reference may also be made to the monitors disclosed in FIG. 1 and FIG. 2. For example, the processor 50 in the monitoring device in FIG. 7 to FIG. 10 may be implemented by the main processor 115 in FIG. 1 and FIG. 2. The physiological sensor 10 may refer to a sensor accessory 111. Moreover, the monitoring device disclosed in FIG. 7 to FIG. 10 may further comprise other components disclosed in FIG. 1 and FIG. 2, for example, the isolated power supply and communication interface 114 and the external communication and power interface 116. Details are not described herein again. In addition, the monitoring device disclosed in FIG. 7 to FIG. 10 may also be all or part of the portable monitoring apparatus 213 shown in FIG. 3. For example, some of the steps in FIG. 4 to FIG. 6 may be performed in the portable monitoring apparatus 213, and some may be performed in the bedside monitor 212. Alternatively, all the steps in FIG. 4 to FIG. 6 are performed in the portable monitoring apparatus 213, and the processed data is displayed, output, and stored by means of the central station 211 or the bedside monitor 212.

The monitoring device disclosed in the disclosure may be a mobile monitoring device, so that the requirements of mobile monitoring can be satisfied without limiting an activity space of a patient. In addition, for a conventional monitoring device, interference may be caused by electrode pulling due to activities such as walking, getting in and out of bed, rubbing clothes by the patient. This may seriously interfere with a waveform signal, and may also affect accuracy of physiological parameter measurement, thereby further affecting the determination of the patient's condition by a doctor and affecting rehabilitation of the patient. In the disclosure, a physiological signal and a motion signal of a patient are acquired synchronously, a signal characteristic of the physiological signal is determined from the physiological signal, and whether the physiological signal is reliable is determined according to the signal characteristic of the physiological signal. If it is determined that the physiological signal is unreliable, a signal characteristic of the motion signal is determined from the motion signal, and a motion state during each heartbeat period of the monitored object is determined according to the signal characteristic of the motion signal. According to the motion state during the heartbeat period, the effectiveness of the physiological signal during the heartbeat period is determined, or the signal characteristic of the physiological signal during the heartbeat period is corrected when a physiological parameter is calculated, thereby satisfying the measurement during mobile monitoring and ensuring measurement performance.

The above description is the method for optimizing physiological parameter using a motion sensor and the monitoring device disclosed in the disclosure, and a practical example is given below for description.

For example, the physiological sensor 10 is an electrocardiogram sensor attached to a monitored object, and an acquired physiological signal is an ECG signal. Therefore, the ECG signal of the monitored object is acquired by means of the electrocardiogram sensor attached to the monitored object, and a motion signal of the monitored object is synchronously acquired by means of a motion sensor attached to the monitored object.

A signal characteristic of the ECG signal is determined from the ECG signal, wherein the signal characteristic of the ECG signal may comprise: an amplitude, slope, width, frequency and the like of a QRS complex, effectiveness and type of the QRS complex, a peak-to-peak interval value, interval value effectiveness, signal quality SQI, and the like.

Whether the ECG signal is reliable is determined according to the signal characteristic of the ECG signal, for example, statistics collection is performed on signal characteristics of the ECG signal, voting and scoring are performed based on different weights of these signal characteristics, a reliability score of the ECG signal is acquired, and whether the ECG signal is reliable is determined based on the acquired reliability score.

When it is determined that the ECG signal is reliable, an ECG parameter such as a heart rate may be directly calculated based on the signal characteristic of the ECG signal.

When it is determined that the ECG signal is unreliable, optimization may be performed according to the motion signal.

Specifically, a signal characteristic of the motion signal is determined from the motion signal, and a motion state during each heartbeat period of the monitored object is determined according to the signal characteristic of the motion signal. For example, the motion state during each heartbeat period of the monitored object is graded according to the signal characteristic of the motion signal, to obtain a motion grade during each heartbeat period of the monitored object. For example, the motion grade may comprise three grades: a low motion grade for indicating that the monitored object has no motion or slight motion, a high motion grade for indicating that the monitored object has intense motion, and a medium motion grade between the low motion grade and the high motion grade.

According to the motion state during the heartbeat period, the effectiveness of the ECG signal during the heartbeat period is determined, or the signal characteristic of the ECG signal during the heartbeat period is corrected when calculating the ECG parameter. Each heartbeat period corresponds to a QRS waveform. For example, when the motion grade is the low motion grade, no extra processing is performed on the ECG signal, and the signal characteristic of the ECG signal may be directly output; when the motion grade is the medium motion grade, a current QRS complex interval of the ECG signal may be corrected to be invalid, the signal quality SQI of the ECG signal may be corrected to 3, and a current QRS complex type of the ECG signal may be determined again; and when the motion grade is the high motion grade, the QRS complex interval of the ECG signal may be directly corrected to be invalid, the signal quality SQI of the ECG signal may be corrected to 4, and the QRS complex type of the electric signal may be corrected to be normal, to reduce the alarm of ventricular arrhythmia.

A person skilled in the art may understand that all or some of the functions of the various methods in the above implementations may be implemented by means of hardware or by means of a computer program. When all or some of the functions in the above implementations are implemented by means of a computer program, the program may be stored in a computer-readable storage medium, and the storage medium may comprise: a read-only memory, a random access memory, a magnetic disk, an optical disk, a hard disk, and the like, and the program is executed by a computer to implement the above functions. For example, the program is stored in a memory of an apparatus, and when the program in the memory is executed by means of a processor, all or some of the above functions can be implemented. In addition, when all or some of the functions in the above implementations are implemented by means of a computer program, the program may also be stored in a storage medium such as a server, another computer, a magnetic disk, an optical disk, a flash disk, or a mobile hard disk, may be saved in a memory of a local apparatus through downloading or copying, or version updating may be performed on a system of the local apparatus. When the program in the memory is executed by means of a processor, all or some of the functions in the above implementations can be implemented.

The disclosure is described by using specific examples above, which are merely for the purpose of facilitating understanding of the disclosure and are not intended to limit the disclosure. For a person of ordinary skill in the art, changes may be made to the above specific implementations according to the idea of the disclosure.

## Claims

1. A computer-implemented method for optimizing physiological parameter using a motion sensor, comprising:
acquiring a physiological signal of a monitored object by a physiological sensor attached to the monitored object, and synchronously acquiring a motion signal of the monitored object by a motion sensor attached to the monitored object; the physiological signal is an ECG signal acquired by an electrocardiogram sensor;
determining a signal characteristic of the physiological signal from the physiological signal, and determining whether the physiological signal is reliable according to the signal characteristic of the physiological signal; wherein the signal characteristic of the physiological signal comprises signal quality, matching of QRS complexes and an effectiveness of QRS complexes, and said determining whether the physiological signal is reliable according to the signal characteristic of the physiological signal comprises: assigning a weight to each signal characteristic of the physiological signal, acquiring a reliability score of the physiological signal through voting and scoring, and determining whether the physiological signal is reliable based on the reliability score;
**characterized in that**,
when determining the physiological signal is reliable, directly calculating the physiological parameter of the monitored object based on the signal characteristic of the physiological signal;
when determining the physiological signal is unreliable, determining a signal characteristic of the motion signal from the motion signal, and determining a motion state by grading the motion comprising at least a low motion grade and a high motion grade, during each heartbeat period of the monitored object during a time period in which the motion signal is acquired according to the signal characteristic of the motion signal, and wherein the heartbeat period is a QRS complex interval; and
according to the motion state during the heartbeat period, determining an effectiveness of the physiological signal during the heartbeat period, wherein the signal is determined as effective based on the low motion grade and ineffective and/or invalid at the high motion grade, or correcting the signal characteristic of the physiological signal based on how the motion has been graded during the heartbeat period when calculating a physiological parameter of the monitored object; and
wherein the signal characteristic of the physiological signal and the motion signal is a time domain characteristic and/or a frequency domain characteristic of a signal; the time domain characteristic of the signal comprises: at least one of a peak position, a peak amplitude, a peak slope, a peak width, peak effectiveness, a peak type, a peak-to-peak interval value, peak-to-peak interval effectiveness, and signal quality.

2. The method of claim 1, further comprising: providing a signal characteristic extraction unit that is configured to extract and output a signal characteristic of an input signal inputted to the signal characteristic extraction unit; wherein the signal characteristic extraction unit is configured to extract and output the signal characteristic of the physiological signal when the input signal is the physiological signal, and extract and output the signal characteristic of the motion signal when the input signal is the motion signal.

3. The method of claim 1, wherein before the signal characteristic of the physiological signal is determined from the physiological signal and the signal characteristic of the motion signal is determined from the motion signal, the method further comprises: preprocessing the physiological signal and the motion signal, wherein the preprocessing the physiological signal and the motion signal comprises: performing at least one of filtering, amplification, and analog-to-digital conversion on the physiological signal and the motion signal.

4. The method of claim 1, wherein said grading the motion state during each heartbeat period of the monitored object during a time period in which the motion signal is acquired according to the signal characteristic of the motion signal, to obtain a motion grade during each heartbeat period of the monitored object comprises: calculating a mean value or a variance of the signal characteristics of the motion signal, and grading the motion state during each heartbeat period of the monitored object during a time period in which the motion signal is acquired through threshold segmentation, to obtain the motion grade during each heartbeat period of the monitored object.

5. The method of claim 1, wherein the motion grade comprises: the low motion grade for indicating that the monitored object has no motion or slight motion, the high motion grade for indicating that the monitored object has intense motion, and a medium motion grade between the low motion grade and the high motion grade.

6. The method of claim 1, wherein said determining an effectiveness of the physiological signal during the heartbeat period, or correcting the signal characteristic of the physiological signal during the heartbeat period when calculating a physiological parameter of the monitored object, according to the motion state during the heartbeat period comprises: determining an effectiveness of the physiological signal according to the motion grade during the heartbeat period, or correcting the signal characteristic of the physiological signal during the heartbeat period according to the motion grade during the heartbeat period when calculating the physiological parameter of the monitored object.

7. The method of claim 6, wherein the signal characteristic comprises signal quality; and said correcting the signal characteristic of the physiological signal during the heartbeat period according to the motion grade during the heartbeat period when calculating the physiological parameter of the monitored object comprises: correcting the signal quality of the physiological signal during the heartbeat period according to the motion grade during the heartbeat period when calculating the physiological parameter of the monitored object.

8. The method of claim 1, said determining an effectiveness of the physiological signal according to the motion grade during the heartbeat period comprises: when the motion grade during the heartbeat period is the low motion grade for indicating that the monitored object has no motion or slight motion, determining that the physiological signal during the heartbeat period is effective; when the motion grade during the heartbeat period is the high motion grade for indicating that the monitored object has intense motion, determining that the physiological signal during the heartbeat period is ineffective; and when the motion grade during the heartbeat period is a medium motion grade between the low motion grade and the high motion grade, reconfirming the effectiveness of the physiological signal during the heartbeat period;
or, said correcting the signal characteristic of the physiological signal during the heartbeat period according to the motion grade during the heartbeat period when calculating the physiological parameter of the monitored object comprises: when the motion grade during the heartbeat period is the low motion grade for indicating that the monitored object has no motion or slight motion, the signal characteristic of the physiological signal is not modified when calculating the physiological parameter of the monitored object; and when the motion grade during the heartbeat period is the high motion grade for indicating that the monitored object has intense motion or a medium motion grade between the low motion grade and the high motion grade, correcting the signal characteristic of the physiological signal when calculating the physiological parameter of the monitored object.

9. The method of claim 1, wherein said correcting the signal characteristic of the physiological signal during the heartbeat period when calculating a physiological parameter of the monitored object comprises: correcting at least one weight of a time domain characteristic or a frequency domain characteristic of the physiological signal during the heartbeat period when calculating the physiological parameter of the monitored object.

10. The method of claim 1,
**characterized in that** when determining the physiological signal is unreliable, the method further comprises: directly calculating the physiological parameter of the monitored object based on the signal characteristic of the physiological signal determined from the physiological signal; and determining whether the calculated physiological parameter during the heartbeat period is effective according to the effectiveness of the physiological signal during the heartbeat period.

11. A monitoring device, comprising:
an electrocardiogram sensor configured to be attached to a monitored object to acquire a physiological signal of the monitored object; the physiological signal is an ECG signal;
a motion sensor configured to be attached to the monitored object to acquire a motion signal of the monitored object; and
a processor configured to: determine a signal characteristic of the physiological signal from the physiological signal, and determine , whether the physiological signal is reliable according to the signal characteristic of the physiological signal, wherein the signal characteristic of the physiological signal comprises signal quality, matching of QRS complexes and an effectiveness of QRC complexes, and said determining whether the physiological signal is reliable according to the signal characteristic of the physiological signal comprises: assigning a weight to each signal characteristic of the physiological signal acquiring a reliability score of the physiological signal through voting and scoring, and determining whether the physiological signal is reliable based on the reliability score;
**characterized in that**, the processor is further configured to:
when determining the physiological signal is reliable, directly calculate the physiological parameter of the monitored object based on the signal characteristic of the physiological signal;
when the physiological signal is unreliable, determine a signal characteristic of the motion signal from the motion signal, and determine a motion state by grading the motion comprising at least a low motion grade and a high motion grade during each heartbeat period of the monitored object during a time period in which the motion signal is acquired according to the signal characteristic of the motion signal, and wherein the heartbeat period is a QRS complex interval; and
according to the motion state during the heartbeat period, determine an effectiveness of the physiological signal during the heartbeat period, wherein the signal is determined as effective based on the low motion grade and ineffective and/or invalid at the high motion grade, or correct the signal characteristic of the physiological signal based on how the motion has been graded during the heartbeat period when calculating a physiological parameter of the monitored object; and
wherein the signal characteristic of the physiological signal and the motion signal is a time domain characteristic and/or a frequency domain characteristic of a signal; the time domain characteristic of the signal comprises: at least one of a peak position, a peak amplitude, a peak slope, a peak width, peak effectiveness, a peak type, a peak-to-peak interval value, peak-to-peak interval effectiveness, and signal quality.

12. The monitoring device of claim 11, further comprises a preprocessing circuit configured to preprocess the physiological signal and the motion signal before the processor determines the signal characteristic of the physiological signal from the physiological signal and determines the signal characteristic of the motion signal from the motion signal, wherein the preprocessing circuit comprises at least one of the following:
a filter circuit configured to filter an input signal;
an amplification circuit configured to amplify an input signal; and
an analog-to-digital conversion circuit configured to perform analog-to-digital conversion on an input signal.

## Patentansprüche

1. Ein computerimplementiertes Verfahren zur Optimierung physiologischer Parameter unter Verwendung eines Bewegungssensors, das Folgendes umfasst:
das Erfassen eines physiologischen Signals des überwachten Patienten durch einen an dem überwachten Patienten angebrachten physiologischen Sensor und das synchrone Erfassen eines Bewegungssignals des überwachten Patienten durch einen an dem überwachten Patienten angebrachten Bewegungssensor; wobei das physiologische Signal ein durch einen Elektrokardiogrammsensor erfasstes EKG-Signal ist;
das Bestimmen einer Signalkennlinie des physiologischen Signals aus dem physiologischen Signal und das Bestimmen, ob das physiologische Signal gemäß der Signalkennlinie des physiologischen Signals zuverlässig ist; wobei die Signalkennlinie des physiologischen Signals die Signalqualität, die Übereinstimmung von QRS-Komplexen und die Wirksamkeit von QRS-Komplexen umfasst, und das Bestimmen, ob das physiologische Signal gemäß der Signalkennlinie des physiologischen Signals zuverlässig ist, Folgendes umfasst: das Zuweisen einer Gewichtung zu jeder Signalkennlinie des physiologischen Signals, das Ermitteln eines Zuverlässigkeitswerts des physiologischen Signals durch Abstimmung und Bewertung sowie das Bestimmen, ob das physiologische Signal zuverlässig ist, auf der Grundlage des Zuverlässigkeitswerts;
**dadurch gekennzeichnet, dass**,
wenn festgestellt wird, dass das physiologische Signal zuverlässig ist, die physiologischen Parameter des überwachten Patienten direkt anhand der Signaleigenschaften des physiologischen Signals berechnet werden;
wenn festgestellt wird, dass das physiologische Signal unzuverlässig ist, eine Signalkennlinie des Bewegungssignals aus dem Bewegungssignal bestimmt wird und ein Bewegungszustand durch Einstufung der Bewegung in mindestens eine niedrige Bewegungsstufe oder eine hohe Bewegungsstufe, während jeder Herzschlagperiode des überwachten Patienten in einem Zeitabschnitt bestimmt wird, in dem das Bewegungssignal gemäß der Signalkennlinie des Bewegungssignals erfasst wird und wobei die Herzschlagperiode ein QRS-Komplex-Intervall ist; und
gemäß dem Bewegungszustand während der Herzschlagperiode eine Wirksamkeit des physiologischen Signals während der Herzschlagperiode bestimmt wird, wobei das Signal auf der Grundlage der geringen Bewegungsstufe als wirksam gilt und als unwirksam und/oder ungültig bei hoher Bewegungsstufe, oder die Signalkennlinie des physiologischen Signals auf der Grundlage der Bewegungseinstufung während des Herzschlagzyklus korrigiert wird, wenn ein physiologischer Parameter des überwachten Patienten berechnet wird; und
wobei die Signalkennlinie des physiologischen Signals und des Bewegungssignals eine Zeitbereichskennlinie und/oder eine Frequenzbereichskennlinie eines Signals ist; die Zeitbereichskennlinie des Signals mindestens eines der folgenden Merkmale umfasst: eine Spitzenposition, eine Spitzenamplitude, eine Spitzensteigung, eine Spitzenbreite, eine Spitzenwirksamkeit, einen Spitzentyp, einen Spitzen-zu-Spitzen-Intervallwert, eine Spitzen-zu-Spitzen-Intervallwirksamkeit und eine Signalqualität.

2. Das Verfahren nach Anspruch 1, das ferner Folgendes umfasst: das Bereitstellen einer Signalkennlinien-Extraktionsvorrichtung, die so ausgelegt ist, dass sie eine Signalkennlinie eines in die Signalkennlinien-Extraktionsvorrichtung eingegebenen Eingangssignals extrahiert und ausgibt; wobei die Signalkennlinien-Extraktionsvorrichtung so ausgelegt ist, dass sie die Signalkennlinie des physiologischen Signals extrahiert und ausgibt, wenn das Eingangssignal das physiologische Signal ist, und die Signalkennlinie des Bewegungssignals extrahiert und ausgibt, wenn das Eingangssignal das Bewegungssignal ist.

3. Das Verfahren nach Anspruch 1, wobei, bevor die Signalkennlinie des physiologischen Signals aus dem physiologischen Signal und die Signalkennlinie des Bewegungssignals aus dem Bewegungssignal ermittelt wird, das Verfahren ferner Folgendes umfasst: das Vorverarbeiten des physiologischen Signals und des Bewegungssignals, wobei das Vorverarbeiten des physiologischen Signals und des Bewegungssignals Folgendes umfasst: das Durchführen mindestens eines der folgenden Schritte: Filtern, Verstärken und Analog-Digital-Wandeln des physiologischen Signals und des Bewegungssignals.

4. Das Verfahren nach Anspruch 1, wobei das Einstufen des Bewegungszustands während jeder Herzschlagperiode des überwachten Patienten während eines Zeitraums, in dem das Bewegungssignal erfasst wird, gemäß den Signaleigenschaften des Bewegungssignals, um eine Bewegungsstufe während jeder Herzschlagperiode des überwachten Patienten zu erhalten, Folgendes umfasst: das Berechnen eines Mittelwerts oder einer Varianz der Signaleigenschaften des Bewegungssignals und das Einstufen des Bewegungszustands während jeder Herzschlagperiode des überwachten Patienten während eines Zeitraums, in dem das Bewegungssignal erfasst wird, mittels Schwellenwertsegmentierung, um die Bewegungsstufe während jeder Herzschlagperiode des überwachten Patienten zu erhalten.

5. Das Verfahren nach Anspruch 1, wobei die Bewegungsstufe Folgendes umfasst: die niedrige Bewegungsstufe, um anzuzeigen, dass der überwachte Patient keine oder nur eine geringe Bewegung zeigt, die hohe Bewegungsstufe, um anzuzeigen, dass der überwachte Patient eine starke Bewegung zeigt, sowie eine mittlere Bewegungsstufe zwischen der niedrigen und der hohen Bewegungsstufe.

6. Das Verfahren nach Anspruch 1, wobei das Bestimmen der Wirksamkeit des physiologischen Signals während der Herzschlagperiode oder das Korrigieren der Signalkennlinie des physiologischen Signals während der Herzschlagperiode bei der Berechnung eines physiologischen Parameters des überwachten Patienten gemäß dem Bewegungszustand während der Herzschlagperiode Folgendes umfasst: das Bestimmen einer Gültigkeit des physiologischen Signals entsprechend der Bewegungsstufe während der Herzschlagperiode oder das Korrigieren der Signalkennlinie des physiologischen Signals während der Herzschlagperiode gemäß der Bewegungsstufe während der Herzschlagperiode bei der Berechnung des physiologischen Parameters des überwachten Patienten.

7. Das Verfahren nach Anspruch 6, wobei die Signaleigenschaft die Signalqualität umfasst; und wobei das Korrigieren der Signaleigenschaft des physiologischen Signals während der Herzschlagperiode entsprechend der Bewegungsstufe während der Herzschlagperiode bei der Berechnung des physiologischen Parameters des überwachten Patienten Folgendes umfasst: das Korrigieren der Signalqualität des physiologischen Signals während der Herzschlagperiode entsprechend der Bewegungsstufe während der Herzschlagperiode bei der Berechnung des physiologischen Parameters des überwachten Patienten.

8. Das Verfahren nach Anspruch 1, wobei das Bestimmen der Wirksamkeit des physiologischen Signals gemäß der Bewegungsstufe während der Herzschlagperiode Folgendes umfasst: wenn die Bewegungsstufe während der Herzschlagperiode die niedrige Bewegungsstufe ist, um anzuzeigen, dass der überwachte Patient keine oder nur eine geringe Bewegung zeigt, wird festgestellt, dass das physiologische Signal während der Herzschlagperiode wirksam ist; wenn die Bewegungsstufe während der Herzschlagperiode die hohe Bewegungsstufe aufweist, um anzuzeigen, dass der überwachte Patient eine intensive Bewegung zeigt, wird festgestellt, dass das physiologische Signal während der Herzschlagperiode unwirksam ist; und wenn die Bewegungsstufe während der Herzschlagperiode eine mittlere Bewegungsstufe zwischen der niedrigen Bewegungsstufe und der hohen Bewegungsstufe aufweist, wird die Wirksamkeit des physiologischen Signals während der Herzschlagperiode erneut bestätigt;
oder wobei das Korrigieren der Signalkennlinie des physiologischen Signals während der Herzschlagperiode entsprechend der Bewegungsstufe während der Herzschlagperiode bei der Berechnung des physiologischen Parameters des überwachten Patienten Folgendes umfasst: wenn die Bewegungsstufe während der Herzschlagperiode die niedrige Bewegungsstufe aufweist, um anzuzeigen, dass der überwachte Patient keine oder nur eine geringe Bewegung zeigt, wird die Signalkennlinie des physiologischen Signals bei der Berechnung des physiologischen Parameters des überwachten Patienten nicht verändert; und wenn die Bewegungsstufe während der Herzschlagperiode die hohe Bewegungsstufe aufweist, um anzuzeigen, dass der überwachte Patient eine starke Bewegung zeigt, oder eine mittlere Bewegungsstufe zwischen der niedrigen und der hohen Bewegungsstufe, die Signalkennlinie des physiologischen Signals bei der Berechnung des physiologischen Parameters des überwachten Patienten korrigiert wird.

9. Das Verfahren nach Anspruch 1, wobei das Korrigieren der Signalkennlinie des physiologischen Signals während der Herzschlagperiode bei der Berechnung eines physiologischen Parameters des überwachten Patienten Folgendes umfasst: das Korrigieren mindestens einer Gewichtung einer Zeitbereichskennlinie oder einer Frequenzbereichskennlinie des physiologischen Signals während der Herzschlagperiode bei der Berechnung des physiologischen Parameters des überwachten Patienten.

10. Das Verfahren nach Anspruch 1,
**dadurch gekennzeichnet, dass**, wenn die Bestimmung des physiologischen Signals unzuverlässig ist, das Verfahren ferner Folgendes umfasst: das direkte Berechnen des physiologischen Parameters des überwachten Patienten auf der Grundlage der Signaleigenschaft des physiologischen Signals, das aus dem physiologischen Signal ermittelt wurde; und das Bestimmen, ob der berechnete physiologische Parameter während der Herzschlagperiode gemäß der Wirksamkeit des physiologischen Signals während der Herzschlagperiode wirksam ist.

11. Eine Überwachungsvorrichtung, die Folgendes umfasst:
einen Elektrokardiogrammsensor, der so ausgelegt ist, dass er an einem überwachten Patienten angebracht wird, um ein physiologisches Signal des überwachten Patienten zu erfassen; wobei das physiologische Signal ein EKG-Signal ist;
einen Bewegungssensor, der so ausgelegt ist, dass er an dem überwachten Patienten angebracht wird, um ein Bewegungssignal des überwachten Patienten zu erfassen; und
ein Prozessor, der dazu ausgelegt ist: eine Signalkennlinie des physiologischen Signals aus dem physiologischen Signal zu ermitteln und anhand der Signalkennlinie des physiologischen Signals zu bestimmen, ob das physiologische Signal zuverlässig ist, wobei die Signalkennlinie des physiologischen Signals die Signalqualität, die Übereinstimmung von QRS-Komplexen und die Wirksamkeit von QRS-Komplexen umfasst, und wobei das Bestimmen, ob das physiologische Signal anhand der Signalkennlinie des physiologischen Signals zuverlässig ist, Folgendes umfasst: das Zuweisen einer Gewichtung zu jeder Signalkennlinie des physiologischen Signals, das Ermitteln eines Zuverlässigkeitswerts des physiologischen Signals durch Abstimmung und Bewertung und das Bestimmen, ob das physiologische Signal zuverlässig ist, basierend auf dem Zuverlässigkeitswert;
**dadurch gekennzeichnet, dass** der Prozessor ferner dazu ausgelegt ist:
wenn festgestellt wird, dass das physiologische Signal zuverlässig ist, den physiologischen Parameter des überwachten Patienten direkt auf der Grundlage der Signaleigenschaften des physiologischen Signals zu berechnen;
wenn das physiologische Signal unzuverlässig ist, eine Signalkennlinie des Bewegungssignals aus dem Bewegungssignal zu bestimmen und einen Bewegungszustand zu bestimmen, indem die Bewegung während jeder Herzschlagperiode des überwachten Patienten in einem Zeitraum, in dem das Bewegungssignal erfasst wird, entsprechend der Signalkennlinie des Bewegungssignals in mindestens eine niedrige Bewegungsstufe und eine hohe Bewegungsstufe eingestuft wird, wobei die Herzschlagperiode ein QRS-Komplex-Intervall ist; und
entsprechend dem Bewegungszustand während der Herzschlagperiode die Wirksamkeit des physiologischen Signals während der Herzschlagperiode zu bestimmen, wobei das Signal bei einer niedrigen Bewegungsstufe als wirksam und bei einer hohen Bewegungsstufe als unwirksam und/oder ungültig eingestuft wird, oder die Signalkennlinie des physiologischen Signals bei der Berechnung eines physiologischen Parameters des überwachten Patienten entsprechend der Einstufung der Bewegung während der Herzschlagperiode korrigiert wird; und
wobei die Signalkennlinie des physiologischen Signals und des Bewegungssignals eine Zeitbereichskennlinie und/oder eine Frequenzbereichskennlinie eines Signals ist; die Zeitbereichskennlinie des Signals mindestens eines der folgenden Merkmale umfasst: eine Spitzenposition, eine Spitzenamplitude, eine Spitzensteigung, eine Spitzenbreite, eine Spitzenwirksamkeit, einen Spitzentyp, einen Spitzen-zu-Spitzen-Intervallwert, eine Spitzen-zu-Spitzen-Intervallwirksamkeit und eine Signalqualität.

12. Die Überwachungsvorrichtung nach Anspruch 11, die ferner eine Vorverarbeitungsschaltung umfasst, die so ausgelegt ist, dass sie das physiologische Signal und das Bewegungssignal vorverarbeitet, bevor der Prozessor die Signalkennlinie des physiologischen Signals aus dem physiologischen Signal und die Signalkennlinie des Bewegungssignals aus dem Bewegungssignal ermittelt, wobei die Vorverarbeitungsschaltung mindestens eines der folgenden Elemente umfasst:
eine Filterschaltung, die so ausgelegt ist, dass sie ein Eingangssignal filtert;
eine Verstärkungsschaltung, die so ausgelegt ist, dass sie ein Eingangssignal verstärkt; und
eine Analog-Digital-Wandlerschaltung, die so ausgelegt ist, dass sie eine Analog-DigitalWandlung an einem Eingangssignal durchführt.

## Revendications

1. Procédé mis en œuvre par ordinateur destiné à optimiser un paramètre physiologique en utilisant un capteur de mouvement, comprenant les étapes consistant à :
acquérir un signal physiologique d'un objet surveillé au moyen d'un capteur physiologique attaché sur l'objet surveillé, et acquérir de manière synchrone un signal de mouvement de l'objet surveillé au moyen d'un capteur de mouvement attaché sur l'objet surveillé ; le signal physiologique est un signal d'ECG acquis au moyen d'un capteur d'électrocardiogramme ;
déterminer une caractéristique de signal du signal physiologique à partir du signal physiologique, et déterminer si le signal physiologique est fiable selon la caractéristique de signal du signal physiologique ; où la caractéristique de signal du signal physiologique comprend une qualité de signal, une correspondance de complexes QRS et une efficacité de complexes QRS, et ledit fait de déterminer si le signal physiologique est fiable selon la caractéristique de signal du signal physiologique comprend : le fait d'attribuer une pondération à chaque caractéristique de signal du signal physiologique, le fait d'acquérir une note de fiabilité du signal physiologique par appréciation et notation, et le fait de déterminer si le signal physiologique est fiable sur la base de la note de fiabilité ;
**caractérisé par**,
lorsqu'il est déterminé que le signal physiologique est fiable, le fait de calculer directement le paramètre physiologique de l'objet surveillé sur la base de la caractéristique de signal du signal physiologique ;
lorsqu'il est déterminé que le signal physiologique n'est pas fiable, le fait de déterminer une caractéristique de signal du signal de mouvement à partir du signal de mouvement, et de déterminer un état de mouvement par classification du mouvement comprenant au moins une classe de faible mouvement et une classe de fort mouvement, durant chaque période de rythme cardiaque de l'objet surveillé durant un intervalle de temps au cours duquel le signal de mouvement est acquis selon la caractéristique de signal du signal de mouvement, et où la période de rythme cardiaque est un intervalle de complexe QRS ; et
selon l'état de mouvement durant la période de rythme cardiaque, le fait de déterminer une efficacité du signal physiologique durant la période de rythme cardiaque, où le signal est déterminé comme efficace sur la base de la classe de faible mouvement et comme inefficace et/ou invalide à la classe de fort mouvement, ou de corriger la caractéristique de signal du signal physiologique sur la base de la manière dont le mouvement a été classé durant la période de rythme cardiaque lors du calcul d'un paramètre physiologique de l'objet surveillé ; et
où la caractéristique de signal du signal physiologique et du signal de mouvement est une caractéristique de domaine temporel et/ou une caractéristique de domaine fréquentiel d'un signal ; la caractéristique de domaine temporel du signal comprend : au moins l'un d'une position de pic, d'une amplitude de pic, d'une pente de pic, d'une largeur de pic, d'une efficacité de pic, d'un type de pic, d'une valeur d'intervalle pic-à-pic, d'une efficacité d'intervalle pic-à-pic, et d'une qualité de signal.

2. Procédé selon la revendication 1, comprenant en outre : le fait de fournir une unité d'extraction de caractéristique de signal qui est configurée pour extraire et fournir une caractéristique de signal d'un signal d'entrée fourni à l'unité d'extraction de caractéristique de signal ; où l'unité d'extraction de caractéristique de signal est configurée pour extraire et fournir la caractéristique de signal du signal physiologique lorsque le signal d'entrée est le signal physiologique, et extraire et fournir la caractéristique de signal du signal de mouvement lorsque le signal d'entrée est le signal de mouvement.

3. Procédé selon la revendication 1, où avant que la caractéristique de signal du signal physiologique ne soit déterminée à partir du signal physiologique et que la caractéristique de signal du signal de mouvement ne soit déterminée à partir du signal de mouvement, le procédé comprend en outre : le fait de prétraiter le signal physiologique et le signal de mouvement, où le fait de prétraiter le signal physiologique et le signal de mouvement comprend : le fait de réaliser au moins l'un d'un filtrage, d'une amplification, et d'une conversion analogique-numérique sur le signal physiologique et le signal de mouvement.

4. Procédé selon la revendication 1, dans lequel ledit fait de classer l'état de mouvement durant chaque période de rythme cardiaque de l'objet surveillé durant un intervalle de temps au cours duquel le signal de mouvement est acquis selon la caractéristique de signal du signal de mouvement, pour obtenir une classe de mouvement durant chaque période de rythme cardiaque de l'objet surveillé comprend : le fait de calculer une valeur moyenne ou une variance des caractéristiques de signal du signal de mouvement, et de classer l'état de mouvement durant chaque période de rythme cardiaque de l'objet surveillé durant un intervalle de temps au cours duquel le signal de mouvement est acquis par segmentation de seuil, pour obtenir la classe de mouvement durant chaque période de rythme cardiaque de l'objet surveillé.

5. Procédé selon la revendication 1, dans lequel la classe de mouvement comprend : la classe de faible mouvement pour indiquer que l'objet surveillé présente peu ou pas de mouvement, la classe de fort mouvement pour indiquer que l'objet surveillé présente un mouvement intense, et une classe de mouvement moyen entre la classe de faible mouvement et la classe de fort mouvement.

6. Procédé selon la revendication 1, dans lequel ledit fait de déterminer une efficacité du signal physiologique durant la période de rythme cardiaque, ou de corriger la caractéristique de signal du signal physiologique durant la période de rythme cardiaque lors du calcul d'un paramètre physiologique de l'objet surveillé, selon l'état de mouvement durant la période de rythme cardiaque comprend : le fait de déterminer une efficacité du signal physiologique selon la classe de mouvement durant la période de rythme cardiaque, ou de corriger la caractéristique de signal du signal physiologique durant la période de rythme cardiaque selon la classe de mouvement durant la période de rythme cardiaque lors du calcul du paramètre physiologique de l'objet surveillé.

7. Procédé selon la revendication 6, dans lequel la caractéristique de signal comprend une qualité de signal ; et ledit fait de corriger la caractéristique de signal du signal physiologique durant la période de rythme cardiaque selon la classe de mouvement durant la période de rythme cardiaque lors du calcul du paramètre physiologique de l'objet surveillé comprend : le fait de corriger la qualité de signal du signal physiologique durant la période de rythme cardiaque selon la classe de mouvement durant la période de rythme cardiaque lors du calcul du paramètre physiologique de l'objet surveillé.

8. Procédé selon la revendication 1, dans lequel ledit fait de déterminer une efficacité du signal physiologique selon la classe de mouvement durant la période de rythme cardiaque comprend : lorsque la classe de mouvement durant la période de rythme cardiaque est la classe de faible mouvement pour indiquer que l'objet surveillé présente peu ou pas de mouvement, le fait de déterminer que le signal physiologique durant la période de rythme cardiaque est efficace ; lorsque la classe de mouvement durant la période de rythme cardiaque est la classe de fort mouvement pour indiquer que l'objet surveillé présente un mouvement intense, le fait de déterminer que le signal physiologique durant la période de rythme cardiaque est inefficace ; et lorsque la classe de mouvement durant la période de rythme cardiaque est la classe de mouvement moyen entre la classe de faible mouvement et la classe de fort mouvement, le fait de reconfirmer l'efficacité du signal physiologique durant la période de rythme cardiaque ;
ou, ledit fait de corriger la caractéristique de signal du signal physiologique durant la période de rythme cardiaque selon la classe de mouvement durant la période de rythme cardiaque lors du calcul du paramètre physiologique de l'objet surveillé comprend : lorsque la classe de mouvement durant la période de rythme cardiaque est la classe de faible mouvement pour indiquer que l'objet surveillé présente peu ou pas de mouvement, la caractéristique de signal du signal physiologique n'est pas modifiée lors du calcul du paramètre physiologique de l'objet surveillé ; et lorsque la classe de mouvement durant la période de rythme cardiaque est la classe de fort mouvement pour indiquer que l'objet surveillé présente un mouvement intense ou la classe de mouvement moyen entre la classe de faible mouvement et la classe de fort mouvement, le fait de corriger la caractéristique de signal du signal physiologique lors du calcul du paramètre physiologique de l'objet surveillé.

9. Procédé selon la revendication 1, dans lequel ledit fait de corriger la caractéristique de signal du signal physiologique durant la période de rythme cardiaque lors du calcul d'un paramètre physiologique de l'objet surveillé comprend : le fait de corriger au moins l'un d'une pondération d'une caractéristique de domaine temporel ou d'une caractéristique de domaine fréquentiel du signal physiologique durant la période de rythme cardiaque lors du calcul du paramètre physiologique de l'objet surveillé.

10. Procédé selon la revendication 1,
**caractérisé en ce que** lorsqu'il est déterminé que le signal physiologique n'est pas fiable, le procédé comprend en outre : le fait de calculer directement le paramètre physiologique de l'objet surveillé sur la base de la caractéristique de signal du signal physiologique déterminée à partir du signal physiologique ; et de déterminer si le paramètre physiologique durant la période de rythme cardiaque est efficace selon l'efficacité du signal physiologique durant la période de rythme cardiaque.

11. Dispositif de surveillance, comprenant :
un capteur d'électrocardiogramme configuré pour être attaché sur un objet surveillé pour acquérir un signal physiologique de l'objet surveillé ; le signal physiologique est un signal d'ECG ;
un capteur de mouvement configuré pour être attaché sur l'objet surveillé pour acquérir un signal de mouvement de l'objet surveillé ; et
un processeur configuré pour : déterminer une caractéristique de signal du signal physiologique à partir du signal physiologique, et déterminer si le signal physiologique est fiable selon la caractéristique de signal du signal physiologique, où la caractéristique de signal du signal physiologique comprend une qualité de signal, une correspondance de complexes QRS et une efficacité de complexes QRS, et ledit fait de déterminer si le signal physiologique est fiable selon la caractéristique de signal du signal physiologique comprend : le fait d'attribuer une pondération à chaque caractéristique de signal du signal physiologique, le fait d'acquérir une note de fiabilité du signal physiologique par appréciation et notation, et le fait de déterminer si le signal physiologique est fiable sur la base de la note de fiabilité ;
**caractérisé en ce que**, le processeur est en outre configuré pour :
lorsqu'il est déterminé que le signal physiologique est fiable, calculer directement le paramètre physiologique de l'objet surveillé sur la base de la caractéristique de signal du signal physiologique ;
lorsqu'il est déterminé que le signal physiologique n'est pas fiable, déterminer une caractéristique de signal du signal de mouvement à partir du signal de mouvement, et déterminer un état de mouvement par classification du mouvement comprenant au moins une classe de faible mouvement et une classe de fort mouvement durant chaque période de rythme cardiaque de l'objet surveillé durant un intervalle de temps au cours duquel le signal de mouvement est acquis selon la caractéristique de signal du signal de mouvement, et où la période de rythme cardiaque est un intervalle de complexe QRS ; et
selon l'état de mouvement durant la période de rythme cardiaque, déterminer une efficacité du signal physiologique durant la période de rythme cardiaque, où le signal est déterminé comme efficace sur la base de la classe de faible mouvement et comme inefficace et/ou invalide à la classe de fort mouvement, ou corriger la caractéristique de signal du signal physiologique sur la base de la manière dont le mouvement a été classé durant la période de rythme cardiaque lors du calcul d'un paramètre physiologique de l'objet surveillé ; et
où la caractéristique de signal du signal physiologique et du signal de mouvement est une caractéristique de domaine temporel et/ou une caractéristique de domaine fréquentiel d'un signal ; la caractéristique de domaine temporel du signal comprend : au moins l'un d'une position de pic, d'une amplitude de pic, d'une pente de pic, d'une largeur de pic, d'une efficacité de pic, d'un type de pic, d'une valeur d'intervalle pic-à-pic, d'une efficacité d'intervalle pic-à-pic, et d'une qualité de signal.

12. Dispositif de surveillance selon la revendication 11, comprenant en outre un circuit de prétraitement configuré pour prétraiter le signal physiologique et le signal de mouvement avant que le processeur ne détermine la caractéristique de signal du signal physiologique à partir du signal physiologique et ne détermine la caractéristique de signal du signal de mouvement à partir du signal de mouvement, où le circuit de prétraitement comprend au moins l'un des éléments suivants :
un circuit de filtre configuré pour filtrer un signal d'entrée ;
un circuit d'amplification configuré pour amplifier un signal d'entrée ; et
un circuit de conversion analogique-numérique configuré pour réaliser une conversion analogique-numérique sur un signal d'entrée.
